# NEUE EUROPÄISCHE PATENTSCHRIFT

(11) **EP 0 901 347 B2**
(45) Veröffentlichungstag und Bekanntmachung des Hinweises auf die Entscheidung über den Einspruch: **13.12.2006**
(45) Hinweis auf die Patenterteilung: 02.10.2002
(21) Anmeldenummer: 97925835.7
(22) Anmeldetag: 07.05.1997
(51) Int. Cl.: A61B 17/28

(54) **INSTRUMENT MIT EINEM ABWINKELBAREN HANDGRIFF**
INSTRUMENT WITH A BENDABLE HANDLE
INSTRUMENT A MANCHE PLIABLE

(30) Priorität: 07.05.1996 DE 19618291
(43) Veröffentlichungstag der Anmeldung: 17.03.1999
(73) Patentinhaber: Karl Storz GmbH & Co. KG, 78532 Tuttlingen (DE)
(72) Erfinder: CUSCHIERI, Alfred, Dundee DD1 9SY (GB); FRANK, Tim, Dundee DD1 9SY (GB)
(74) Vertreter: Meissner, Bolte & Partner
(86) Internationale Anmeldenummer: PCT/DE1997/000910
(87) Internationale Veröffentlichungsnummer: WO 1997/041783

(56) Entgegenhaltungen:
- WO-A-95/10230
- DE-A- 4 104 755
- US-A- 4 950 273
- US-A- 5 094 247
- US-A- 5 234 460
- US-A- 5 395 367

## Beschreibung

### Technisches Gebiet

Die Erfindung bezieht sich auf ein Instrument gemäß dem Oberbegriff des Patentanspruchs 1.

### Stand der Technik

Instrumente, die ein Schaftteil aufweisen, an dessen distalem Ende ein Eingriffselement angeordnet ist, das durch ein proximal angeordnetes Betätigungselement betätigt wird, das in einen Handgriff, integriert ist, sind im Bereich der Medizintechnik und insbesondere der Endoskopie allgemein bekannt. Nur beispielhaft wird auf Rohrschaft-Instrumente der Karl Storz GmbH & Co., Tuttlingen, Deutschland verwiesen.
Bei den meisten bekannten Instrumenten ist der Handgriff fest an dem Instrument angebracht, so daß die Achse des Handgriffs einen vorgegebenen, unveränderbaren Winkel mit der Längsachse des Schafts des Instruments einschließt. Dieser Winkel ist unter ergonomischen Gesichtspunkten für eine bestimmte Handhabung des Instruments gewählt.

Aus der Druckschrift US-A-5 094 247 ist ein Instrument mit einem abwinkelbaren Handgriff bekannt, von dem bei der Bildung des Oberbegriffs des Patentanspruchs 1 ausgegangen wurde. In der US-A-5 094 247 ist ein Instrument mit einem Schaftteil offenbart, an dessen distalem Ende ein Eingriffselement angeordnet ist, welches durch ein proximal angeordnetes Betätigungselement betätigt wird, das in einen Handgriff integriert ist, der über eine Kardangelenk mit dem Schaftteil verbunden ist, so dass er derart bewegbar ist, dass der von der Achse des Handgriffs und der Achse des Schaftteils eingeschlossene Winkel variierbar ist.

Aus der US-A-5 395 367 ist ein Instrument bekannt, dass dadurch gekennzeichnet ist, dass das Verbindungselement in Form eines Balges bzw. eines akkordeonähnlichen Elements ausgebildet ist. Ähnlich einem Trinkstrohhalm für Kinder lässt sich dieses Element in einer Vielzahl von unterschiedlichen Winkeln einstellen. Die Einstellung ist jedoch nicht gegen unbeabsichtigtes Verstellen z.B. während des Operationsvorgangs sicherbar. Vielmehr lässt sich das Verbindungselement (der "Balg") wie auch beim Trinkstrohhalm leicht verstellen.
Die bisher bekannten Lösungen zeigen, dass die Idee, ein medizinisches Instrument zu schaffen, bei dem der Handgriff beliebig abwinkelbar ist, schon sehr alt ist. Dennoch sind keine derartigen Instrumente auf den Markt gekommen. Der Grund dafür ist nach diesseitiger Auffassung, dass bei allen Vorschlägen bisher übersehen worden ist, dass eine freie Einstellbarkeit der Winkelstellung des Handgriffs zugleich auch die Möglichkeit beinhaltet, den Handgriff während des Operationsvorgangs unbeabsichtigt zu verstellen.
Die Möglichkeit der Feststellung in einer beliebigen gewünschten Stellung ist in keinem dieser hier nur beispielhaft aufgeführten Werke genannt. Insbesondere sind jedoch keine medizinischen Instrumente mit einem Kardangelenk bekannt, wo die Fixierung des Gelenkes in einer bestimmten Stellung beschrieben ist.

Zur Lösung der erfindungsgemäß gestellten Aufgabe wird von einem Instrument ausgegangen, bei dem der Handgriff in einer bestimmten Winkelstellung bzw. bei Verdrehbarkeit um die Längsachse auch bei einem bestimmten Drehwinkel feststellbar ist.

Erfindungsgemäß ist der Handgriff über ein Kardangelenk mit dem Schaftteil verbunden. Die Verwendung eines Kardangelenks hat den Vorteil, daß das Kardangelenk auch unter Operationsbedingungen und dem damit verbundenen Streß leicht eingestellt werden kann, so daß eine optimale Einstellbarkeit der Stellung des Handgriffs relativ zum Schaft gegeben ist. Darüberhinaus erlaubt ein Kardangelenkt die Einstellung der Lage des Handgriffs in "alle Richtungen" bezogen auf den Schaft des Instruments, so daß nicht nur die Winkelstellung geändert werden kann.

Damit kann je nach Bedienungsbedingung, wie sie sich durch die jeweilige operationsbedingte Handhabung ergibt, die Stellung des Handgriffs relativ zur Längsachse des Schaftteils geeignet eingestellt werden, so daß der Operateur das Instrument unabhängig vom jeweiligen Einsatzfall ergonomisch günstig bedienen kann.

Insbesondere ist es damit möglich , den Handgriff so anzuordnen, daß seine Achse einen Winkel zwischen 0° und 90° mit der Längsachse des Schaftteils einschließt.

Bei einer Weiterbildung kann der Handgriff aufgrund der Ausbildung des Kardangelenks um die Längsachse des Schaftteils gedreht werden. Die Relatiwerdrehung zwischen Handgriff und Schaft ist zwar bereits bei bestimmten Instrumenten realisiert, in Verbindung mit der erindungsgemäß vorgesehenen Einstellung des Winkels zwischen Handgriff und Schaft hat sie jedoch besondere ergonomische Vorteile.

Die erfindungsgemäße Winkeleinstellung ermöglicht in jeder Operationssituation ein ergonomische Arbeiten. In bestimmten Situationen kann es jedoch von Nachteil sein, wenn sich die Relativstellung des Handgriffs zum Schaft des Instruments aufgrund der Verwendung eines prinzipiell leicht einstellbaren Kardangelenks "zu leicht" ändern läßt.

Zur ergonomischen Bedienbarkeit der Erfindung trägt weiterhin bei, wenn ein flexibles Kraftübertragungselement vorgesehen ist, das die Betätigungskraft vom Betätigungselement auf das Schaftteil überträgt. Das Kraftübertragungselement kann insbesondere ein Bowdenzug sein.

Der erfindungsgemäße Grundgedanke, den Winkel, der zwischen der Achse des Schafts, d.h. der Längsachse des Instruments und der Achse des Handgriffs eingeschlossen ist, frei auch während einer Operation variieren zu können, läßt sich prinzipiell bei den verschiedensten Instrumenten anwenden. Besonders vorteilhaft ist die Anwendung dieses Grundgedankens jedoch bei Rohrschaftinstrumenten sowie bei Instrumenten, wie sie in der Endoskopie eingesetzt werden, also bei Instrumenten, die in den Kanal eines endoskopischen Einsetzinistruments, wie einer Trokarhülse einsetzbar sind.

### Kurze Beschreibung der Zeichnung

Die Erfindung wird nachstehend ohne Beschränkung des allgemeinen Erfindungsgedankens anhand eines Ausführungsbeispiels unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird.
Es zeigen:
- Fig. 1: ein Ausführungsbeispiel eines erfindungsgemäßen Instruments im nichtbetätigten Zustand,
- Fig. 2: das in Fig. 1 gezeigte Ausführungsbeispiel im betätigten Zustand, und
- Fig. 3: das in Fig. 1 gezeigten Ausführungsbeispiel in einem Zustand, in dem der Handgriff um 90° abgewinkelt ist.

### Beschreibung eines Ausführungsbeispiels

In den Figuren ist ein Ausführungsbeispiel eines erfindungsgemäßen Instruments dargestellt. Das Instrument weist einen Schaftteil 1 auf, an dessen distalem Ende ein Eingriffselement 2 angeordnet ist. Bei dem gezeigten Ausführungsbeispiel ist das Eingriffselement 2 ohne Beschränkung des allgemeinen Erfindungsgedankens ein Zangenmaul. Das Eingriffselement 2, also das Zangenmaul wird durch ein proximal angeordnetes Betätigungselement betätigt.

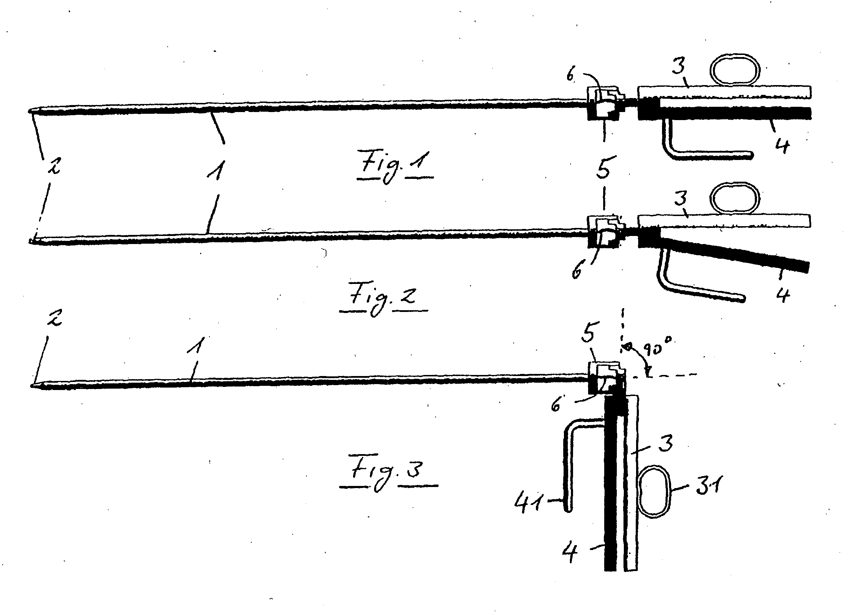

Fig. 1 zeigt das Zangenmaul im geschlossenen Zustand, während Fig. 2 das Zangenmaul im geöffneten Zustand zeigt.

Bei dem gezeigten Ausführungsbeispiel ist das Betätigungselement ein aus zwei Handstücken 3 und 4 bestehender Handgriff. Das Handstück 3 ist - bezogen auf den Handgriff als Einheit - feststehend und weist einen "Fingerring" 31 auf, der typischerweise von dem Daumen der Bedienungsperson durchsetzt wird. Das bewegliche Handstück 4 weist bei dem gezeigten Ausführungsbeispiel einen Fingerbügel 41 auf, der ein Öffnen des Zangenmauls 2 ermöglicht, und beim Schließen ein Abrutschen des an ihm anliegenden Fingers, also typischerweise des Zeigefingers oder des M ittelfingers verhindert.

Der aus den Handstücken 3 und 4 bestehende Handgriff ist bei dem gezeigten Ausführungsbeispiel über ein allseits bewegliches Kardangelenk 5 mit dem Schaftteil 1 verbunden.

Durch dieses Kardangelenk 5 ist der Handgriff 3,4 an dem Schaftteil 1 derart bewegbar angeordnet, daß der von der Achse des Handgriffs 3,4 und der Achse des Schaftteils 1 eingeschlossene Winkel variiert werden kann. In den Figuren 1 und 2 ist der Winkel 0°, während in Fig. 3 der Winkel 90° ist.

Da das Gelenk 5 ein allseits bewegliches Kardangelenk ist, kann der Handgriff 3,4 zusätzlich um die Längsachse des Schaftteils 1 gedreht werden kann, so daß der Handgriff in beliebige Winkelstellung gebracht werden kann, ohne daß der Schaft gedreht werden müßte.

Damit ist es möglich, den Schaft des Instruments so einzurichten und insbesondere so zu drehen, daß sich das Zangerunaul in der gewünschten Stellung relativ zum Gewebe befindet, und gleichzeitig den Handgriff in eine ergonomisch günstige Stellung zu bringen, in der beispielsweise die Bedienungsperson leicht die erforderliche Kraftfüreinen Schnitt aufbringen kann.

In dem Gelenk 5 ist ein flexibles Kraftübertragungselement 6 vorgesehen, das die Betätigungskraft, die von den Handstücken 3 und 4 ausgeübt wird, auf das Schaftteil 1 überträgt. Dieses flexible Kraftübertragungselement ist bevorzugt ein Bowdenzug, der im Zusammenwirken mit dem universell einstellbaren Kardangelenk der Bedienungsperson alle Freiheiten bei der Einstellung der Orientierung des Handgriffs bezüglich seiner Winkellage zur Längsachse und seiner Drehstellung um die Längsachse gibt.

Der Handgriff ist in einer bestimmten Winkelstellung bzw. Drehstellung feststellbar. Dies kann beispielsweise durch eine Feststellschraube erfolgen, die das Gelenk 5 arretiert. Die Schraube ist bevorzugt so ausgebildet, daß sie während eines Operationsvorganges auch ohne Werkzeuge geöffnet und wieder angezogen werden kann.

Vorstehend ist die Erfindung anhand eines Ausführungsbeispiels beschrieben worden. Selbstverständlich können die verschiedensten Abwandlungen vorgenommen werden:

Anstelle des in den Figuren dargestellten Rohrschaftinstruments können auch andere Instrumente, wie beispielsweise HF-Instrumente eingesetzt werden. Besonders bevorzugt ist es jedoch, wenn das jeweilige Instrument derart ausgebildet ist, daß es in den Kanal eines endoskopischen Einsetzinstruments, wie einer Trokarhülse einsetzbar ist.

## Patentansprüche

1. Instrument mit einem Schaftteil (1), an dessen distalem Ende ein Eingriffselement (2) angeordnet ist, das durch ein proximal angeordnetes Betätigungselement betätigt wird, das in einen Handgriff (3, 4) integriert ist, der über ein Kardangelenk (5) mit dem Schaftteil (1) verbunden ist, so dass er derart bewegbar ist, dass der von der Achse des Handgriffs (3, 4) und der Achse des Schaftteils (1) eingeschlossene Winkel variiert werden kann,
**dadurch gekennzeichnet, dass** der Handgriff (3, 4) in einer bestimmten Stellung **dadurch** feststellbar ist, dass die Bewegungen des Kardangelenks (5) blockiert werden wozu eine Feststellschraube am Kardangelenk (5) vorgesehen ist, die durch Feststellen die Bewegungen des Kardangelenks (5) blockiert.

2. Instrument nach Anspruch 1,
**dadurch gekennzeichnet, dass** ein flexibles Kraftübertragungselement (6) vorgesehen ist, das die Betätigungskraft vom Betätigungselement auf das Schaftteil (1) überträgt.

3. Instrument nach einem der Ansprüche 1 oder 2,
**dadurch gekennzeichnet, dass** der Handgriff (3, 4) um die Längsachse des Schaftteils (1) drehbar ausgebildet ist.

4. Instrument nach einem der Ansprüche 1 bis 3,
**dadurch gekennzeichnet, dass** das Instrument ein Rohrschaftinstrument ist.

5. Instrument nach einem der Ansprüche 1 bis 4,
**dadurch gekennzeichnet, dass** das Instrument derart ausgebildet ist, dass es in den Kanal eines endoskopischen Einsetzinstruments einsetzbar ist.

## Claims

1. Instrument having a shaft part (1), on the distal end of which there is disposed an operational element (2) which is actuated by a proximally disposed actuating element which is integrated into a handle (3, 4) which is connected to said shaft part (1) via a Cardan joint (5) so that it can be moved in such a way that the angle formed by the axis of the handle (3, 4) and the axis of the shaft part (1) can be varied,
**characterised in that** the handle (3, 4) can be locked in a certain position through the fact that the movements of the Cardan joint (5) are jammed, for which purpose there is provided on said Cardan joint (5) a locking screw which jams the movements of the latter by locking them.

2. Instrument according to Claim 1,
**characterised in that** a flexible force-transmitting element (6) is provided, which transmits the actuating force from the actuating element to the shaft part (1).

3. Instrument according to one of Claims 1 or 2,
**characterised in that** the handle (3, 4) is constructed so as to be rotatable about the longitudinal axis of the shaft part (1).

4. Instrument according to one of Claims 1 to 3,
**characterised in that** the instrument is a tubular-shaft instrument.

5. Instrument according to one of Claims 1 to 4,
**characterised in that** the instrument is constructed in such a way that it can be inserted in the duct of an endoscopic insertable instrument

## Revendications

1. Instrument comprenant un élément de hampe (1) sur une extrémité distale duquel est disposé un élément opérationnel (2) qui est actionné par un élément manipulateur disposé du côté proximal, lequel est intégré dans une poignée (3, 4) reliée audit élément de hampe (1) par l'intermédiaire d'un joint de cardan (5) d'une façon mobile, de telle sorte que l'angle formé par l'axe de ladite poignée (3, 4) et par l'axe dudit élément de hampe (1) soit variable,
**caractérisé en ce que** la poignée (3, 4) est apte à être arrêtée dans une position déterminée, de telle sorte que les mouvements du joint à cardan (5) sont bloqués, une vis de serrage étant disposée sur ledit joint de cardan (5) pour bloquer les mouvements dudit joint de cardan (5) par serrage.

2. Instrument selon la revendication 1,
**caractérisé en ce qu'**il est prévu un élément flexible de transmission de force (6) qui transfère la force d'actionnement depuis l'élément manipulateur audit élément de hampe (1).

3. Instrument selon la revendication 1 ou 2,
**caractérisé en ce que** la poignée (3, 4) est agencée de façon rotative autour de l'axe longitudinal dudit élément de hampe (1).

4. Instrument selon l'une des revendications 1 à 3,
**caractérisé en ce que** l'instrument est un instrument à hampe tubulaire.

5. Instrument selon l'une quelconque des revendications 1 à 4,
**caractérisé en ce que** l'instrument est configuré de telle sorte qu'il est apte à être introduit dans le canal d'un instrument d'insertion endoscopique.
